# EUROPEAN PATENT SPECIFICATION

(11) **EP 4 029 938 B1**
(45) Date of publication and mention of the grant of the patent: **28.01.2026**
(21) Application number: 20862595.4
(22) Date of filing: 11.09.2020
(51) Int. Cl.: C12Q 1/6806, C12N 15/09, C12Q 1/6851, C12Q 1/686, C12Q 1/6876, G01N 33/50

(54) **NUCLEIC ACID DETECTION METHOD BY REAL-TIME PCR**
VERFAHREN ZUR DETEKTION VON NUKLEINSÄUREN MITTELS ECHTZEIT-PCR
MÉTHODE DE DÉTECTION D'ACIDE NUCLÉIQUE PAR PCR EN TEMPS RÉEL

(30) Priority: 12.09.2019 JP 2019166510
(43) Date of publication of application: 20.07.2022
(73) Proprietor: Sekisui Medical Co., Ltd., Tokyo 103-0027 (JP); National Center for Child Health and Development, Tokyo 157-8535 (JP)
(72) Inventor: EBINUMA, Hiroyuki, Tokyo 103-0027 (JP); INOUE, Hiroaki, Tokyo 103-0027 (JP); UCHIYAMA, Toru, Tokyo 157-8535 (JP)
(74) Representative: Hoffmann Eitle
(86) International application number: PCT/JP2020/034398
(87) International publication number: WO 2021/049601

(56) References cited:
- WO-A1-2018/181850
- WO-A1-2018/181850
- WO-A1-2019/044952
- WO-A1-2019/044952
- WO-A1-2019/074004
- JP-A- 2004 283 165
- JP-A- 2011 019 505
- JP-A- 2011 019 505
- JP-A- 2012 157 295

## Description

### TECHNICAL FIELD

The present invention relates to a method for detecting a nucleic acid by real-time PCR. Specifically, the invention relates to a method for optically detecting a target nucleic acid in a blood spot in a filter paper obtained by blotting blood on the filter paper and then drying the blood, a nucleic acid quantification method by the detection method and the use of a quantification kit in the quantification method.

### BACKGROUND ART

As an analyte for screening and diagnosing a disease, a blood spot in a filter paper obtained by blotting blood on the filter paper and then drying the blood is useful in terms of the storage and the transportation because the sample is highly stable and because there is no possibility of scattering. Moreover, a blood spot in a filter paper is used in newborn mass screening as a method for collecting an analyte from a newborn, from which normal blood collection is difficult. For the purpose of screening and diagnosing a disease, PCR reaction is widely used for the determination of presence or absence of a mutation in a related gene or for the quantification of a gene. Specifically, as a screening test, quantification of circular DNA TRECs (T-cell receptor excision circles) as an indicator of severe combined immunodeficiency and circular DNA KRECs (kappa-deleting recombination excision circles) as an indicator of X-linked agammaglobulinemia is generally used mainly in the U.S. Moreover, since a therapeutic agent for spinal muscular atrophy has been developed, there is a growing demand for screening by homozygous deletion of *SMN1* (Survival Motor Neuron 1) gene, which causes therefor.

Because substances that inhibit PCR reaction (hemoglobin and the like) are contained in a high amount in a dried blood spot in a filter paper used for the test, in a generally used method, nucleic acid is extracted from the blood spot in a filter paper and purified to obtain a reaction sample. In this regard, a reagent which reduces the influence of the substances inhibiting PCR reaction due to the contaminants in the analyte is commercially used (manufactured by Shimadzu Corporation; Ampdirect (registered trademark)). When the reagent is used, PCR reaction can be performed directly using a piece of a blood spot in a filter paper, and the amplification product is observed by detection with electrophoresis (Patent Document 1). With such a reagent, however, in the case of detection using optical means, hemoglobin contained in the blood and precipitates of blood components generated by thermal denaturation during the PCR reaction block the optical path, and thus accurate results cannot be obtained. Accordingly, a method in which the ratio of the whole blood sample added to the reaction solution is defined for the PCR reaction is disclosed (Patent Document 2). However, in addition to the influence derived from the whole blood components, the filter paper piece derived from the dried blood spot in a filter paper which is present in the PCR reaction solution itself sometimes blocks the light path of the optical detection, and thus the influence may not be reduced sufficiently.

In a method conducted to solve the problems, a simple pretreatment step, such as washing of the blood spot in a filter paper piece, immersion in a pretreatment solution and immersion in a pretreatment solution and warming, is conducted to remove the substances which inhibit the reaction and to prevent the diffusion of the substances in the reaction solution, and after the completion of the pretreatment step, PCR reaction is performed without removing the blood spot in a filter paper piece after adding the PCR reaction reagent (Non-Patent Document 1).

However, the method cannot be considered as the best method because the possibility of contamination is higher due to the necessity for multiple-stage operations and because the analysis costs increase due to the required time and work for the analysis of multiple analytes during newborn screening and the like.

Moreover, in a possible method, a calibrator (standard) of the blood spot specimen base may be set to offset the influence of the blood spot in a filter paper for quantification, but the accuracy and the reproducibility may be deteriorated by the variation in the degree of influence due to the variation in the components extracted from the blood spot in a filter paper and the like.

WO2018/181850 discloses a method for detecting a target nucleic acid contained in a dried blood spot in a filter paper by real-time PCR. However, WO2018/181850 does not disclose a detection method which does not rely on a pre-treatment.

### CITATION LIST

### PATENT LITERATURE

Patent Document 1: JP-A-2004-283165
Patent Document 2: JP 5144518 B

### NON-PATENT LITERATURE

Non-Patent Document 1: Clinical Chemistry, 61:2, 412-419, 2015

### SUMMARY OF INVENTION

### TECHNICAL PROBLEM

An object of the invention is to provide an optical detection method and a quantification method of a product obtained by amplifying a target nucleic acid contained in a piece of a blood spot in a filter paper obtained by blotting blood on the filter paper and then drying the blood using nucleic acid amplification reaction by real-time PCR and to further provide the use of a quantification kit in the detection and quantification methods.

### SOLUTION TO PROBLEM

A dried blood spot in a filter paper is obtained by blotting blood on a filter paper and then drying the blood and taken out using a punching device as a paper piece having the shape of the punching hole (sometimes simply referred to as a punched piece in the present specification). As a result of investigations of the size of such a punched piece or the whole blood amount contained in the punched piece, the amount of the PCR reaction reagent, capping of the PCR reaction tube with a cap during the PCR reaction and the like, the present inventors have found that a target nucleic acid can be optically detected and quantified directly from a dried blood spot in a filter paper by real-time PCR without the conventional complicated pretreatment. The invention has been thus completed.

That is, the invention has the following structures.
<1> A method for detecting a target nucleic acid contained in a dried blood spot in a filter paper by real-time PCR, including the steps of (A) to (D) below:
   (A) a step of adding the dried blood spot in a filter paper to a PCR reaction tube, wherein the filter paper is a circular punched piece with a diameter of 1.2 mm to 2.0 mm or a punched piece containing whole blood in an amount of 0.95 v/v% to 6.6 v/v% based on the total amount of the reaction solution;
   (B) a step of adding 20 to 50 µL of a PCR reagent to the PCR reaction tube;
   (C) a step of performing PCR reaction in the tube which contains the PCR reagent and the dried blood spot in a filter paper and which is sealed with a cap; and
   (D) a step of sequentially and optically detecting the target nucleic acid amplified by the PCR reaction.
<2> The method for detecting a target nucleic acid described in <1>, wherein the step of (B) is conducted after the step of (A).
<3> The method for detecting a target nucleic acid described in <1> or <2>, wherein the PCR reaction tube is a 96-well plate for PCR reaction or a tube in an eight-tube strip.
<4> The method for detecting a target nucleic acid described in any of <1> to <3>, wherein the PCR reagent contains at least a primer, a polymerase, dNTPs and an intercalator or a fluorescently labeled probe.
<5> The method for detecting a target nucleic acid described in <4>, wherein the amplified target nucleic acid is detected through detection of fluorescence emitted by irradiation of the intercalator with an excitation light.
<6> The method for detecting a target nucleic acid described in <4>, wherein the fluorescently labeled probe has a fluorescent substance and a quencher and includes a partial sequence complementary to a template of the nucleic acid amplification reaction, and the amplified target nucleic acid is detected through detection of fluorescence emitted by irradiation of the fluorescent substance with an excitation light.
<7> The method for detecting a target nucleic acid described in any of <1> to <6>, wherein the target nucleic acid is one or more gene fragments and/or genes selected from the group consisting of TRECs, KRECs and *SMN1.*
<8> A method for quantifying a target nucleic acid by the method for detecting a target nucleic acid by real-time PCR described in any of <1> to <7>, wherein the target nucleic acid is quantified using a standard below;
   (1) a standard which contains an artificial nucleic acid including the sequence of the target nucleic acid and which is not contained in the dried blood spot in a filter paper, wherein the standard is dissolved directly in a reaction container and used.
<9> The use of a quantification kit used for a method for quantifying a target nucleic acid by the method for detecting a target nucleic acid by real-time PCR described in any of <1> to <7>, including at least the following;
   (1) a standard which contains an artificial nucleic acid including the sequence of the target nucleic acid and which is not contained in the dried blood spot in a filter paper, wherein the standard is dissolved directly in a reaction container and used.

### ADVANTAGEOUS EFFECTS OF INVENTION

According to the invention, specific and rapid detection and quantification of a slight amount of a target nucleic acid contained in a dried blood spot in a filter paper by direct real-time PCR are enabled. For example, in a screening test of newborns for TRECs, KRECs, *SMN1* or *SMN2,* a part of a dried blood spot in a filter paper is taken out as a punched piece and used as an analyte, and thus this leads to the saving of the valuable analyte. Moreover, because no pretreatment step is necessary, the improvement of the analysis efficiency as a screening test and the reduction in costs are possible.

### DESCRIPTION OF EMBODIMENTS

### (PCR Reaction Tube)

The tube used in the invention is a tube for PCR reaction and has a structure into which a punched piece of the blood spot in a filter paper can be smoothly inserted and which can be sealed with a cap. In a suitable shape, the opening at the upper part of the tube is a circle (for example, an inside diameter of around 2.5 mm to 10 mm), and the bottom is narrower than the opening at the upper part. An inverted cone shape or the like is preferable.

The volume of the tube may be a volume to which the PCR reaction reagent can be added and which can sufficiently hold the reaction solution even with the temperature changes during the PCR reaction, and the tube preferably has a volume of 0.1 mL to 0.3 mL, further preferably a volume of 0.15 mL to 0.25 mL, most preferably a volume of 0.2 mL.

The PCR reaction tube used in the invention is preferably a 96-well plate for PCR reaction in which 96 plates are connected or one in an eight-tube strip in which eight tubes are connected, and as commercial products, those sold with a name 96-well PCR plate, PCR eight-tube strip or the like can be used. Commercial products include LightCycler 480 Multiwell Plate 96 (Roche, Inc.), 96-Well PCR Plate, Flat Top, Low Profile, Natural, Polypropylene, UltraFlux (Scientific Specialties, Inc.), Eppendorf PCR plate 96, skirtless, 150 µL, colorless (Eppendorf), PCR plate 96-well simplate 0.1 ml natural (BM Equipment Co., Ltd.), LightCycler (registered trademark) 8-Tube Strips (Roche, Inc.) and the like.

The material of such a reaction tube desirably causes little contamination of the reaction solution and is stiff so that the shape does not change even with the changes in the internal pressure due to the temperature changes during the PCR reaction, and the tube is made of polypropylene, for example.

### (Cap)

The cap used in the invention has a structure which can fit and seal the opening of the PCR reaction tube. Like the PCR reaction tube, the material is desirably a material which does not deform with the changes in the internal pressure due to the PCR reaction and is desirably a highly optically transparent material, and the cap may be made of polypropylene. Specific examples thereof include LightCycler (registered trademark) 8-Tube Strips (Roche, Inc.), optical flat 8-cap strips (Bio-Rad Laboratories, Inc.), MicroAmp^{™} Optical 8-Cap Strips (Thermo Fisher Scientific), Strips of 8 Flat Optical Caps (NIPPON Genetics Co, Ltd), cap strips, 8-strips, flat (Eppendorf) and the like.

In the invention, when the PCR reaction is performed in the PCR reaction tube which is sealed with the cap, generation of bubbles due to the thermal cycles can be prevented. As a result, vibration of the punched piece in the reaction solution and lifting of the punched piece are prevented. Therefore, excessive elution of interfering substances contained in the punched piece into the reaction solution is thought to be prevented. Moreover, because lifting of the punched piece due to generation of bubbles can be prevented, the optical detection by real-time PCR can be conducted with excellent reproducibility without blocking of the light path by the punched piece.

### (Blood Sample/Dried Blood Spot in a Filter Paper)

The blood sample used in the invention is blood (whole blood) in a filter paper that is obtained by blotting blood (whole blood) on the filter paper and then drying the blood and is sometimes simply called a dried blood spot in a filter paper. The dried blood spot in a filter paper is taken out using a punching device as a paper piece having the shape of the punching hole (punched piece). The size of the punched piece is desirably 1.2 to 2.0 mm in diameter, further desirably 1.5 mm to 1.8 mm, because the amount of the contained blood should be in an appropriated range. The whole blood amount contained in the punched piece based on the total amount of the PCR reaction solution is desirably 0.95 v/v% to 6.6 v/v%, further desirably 1.4 v/v% to 5.2 v/v%. The whole blood amount contained in the punched piece based on the total amount of the PCR reaction solution is desirably 0.95 v/v% to 6.6 v/v%, further desirably 1.4 v/v% to 5.2 v/v%. Here, the whole blood amount is indicated as the proportion which is obtained by adding a circular punched piece with a diameter of 1.2 to 2.0 mm or a punched piece having a diameter of 1.5 mm to 1.8 mm to 20 to 50 µL of a PCR reaction solution and dissolving the blood and which is calculated based on the actual value (when 30 µL of whole blood was added to a filter paper before cutting out a punched piece and dried, the diameter of the created circular blood portion was of 9.5 mm).

Examples of the dried blood spot in a filter paper used in the invention include a blood spot in a filter paper collected from the heel of a newborn during newborn mass screening or the like, a blood spot in a filter paper collected during simultaneous optional screening and the like, but the dried blood spot in a filter paper is not limited to the examples.

### (PCR Reagent)

The PCR reagent used in the invention contains at least a set of primers, a polymerase, dNTPs (deoxynucleoside triphosphates), an intercalator or a fluorescently labeled probe. The reagent also typically contains a buffer. The buffer is not particularly limited as long as the buffer has the function of inhibiting the activities of substances which inhibit the DNA amplification reaction, such as positively charged substances (certain kinds of protein and the like) and negatively charged substances (certain kinds of saccharide, dyes and the like), in the body fluid of a living thing. Examples of commercial buffers include Ampdirect, Ampdirect plus (both manufactured by Shimadzu Corporation) and the like.

The amount of the PCR reagent used in the invention added to a reaction tube is 20 to 50 µL.

### (Real-Time PCR)

The real-time PCR method generally means a method for sequentially monitoring the process of generation of an amplification product in PCR. In the invention, detection by real-time PCR means detection of an amplified target nucleic acid (also simply referred to as an amplification product below) by optical means after the PCR reaction. Here, the amplification product may be detected, for example, at the time of completion of the PCR reaction or after the completion or may be detected simultaneously during the PCR reaction step. In the case of simultaneous detection, the amplification product can be detected, for example, sequentially. The sequential detection (monitoring) may be, for example, continuous or noncontinuous (intermittent) detection.

The temperature changes of the steps in the PCR reaction may be automatically controlled using, for example, a thermal cycler or the like. In the PCR reaction in the invention, because the PCR reaction is started after sealing the tube with the cap, generation of bubbles due to the temperature changes can be prevented, and vibration can be prevented without lifting the punched piece. As a result, excessive elution of interfering substances from the punched piece can be prevented, and optical detection and quantification of the target nucleic acid contained in the punched piece have been enabled without conducting any complicated pretreatment. Moreover, during real-time PCR, which is continuous monitoring of PCR reaction, the punched piece cannot be forcibly placed at the bottom by centrifugation or the like in the middle of the reaction, and thus lifting of the punched piece is fatal for the optical detection. Because lifting of the punched piece can be prevented by the invention, continuous optical detection by real-time PCR without blocking of the light path by the punched piece has been enabled.

The amplification product generated by the PCR reaction can be detected, for example, by detecting the fluorescence intensity generated from the amplification product. The fluorescence detection is not particularly restricted but is the conventionally known intercalator method, the TaqMan (registered trademark) probe method, the hybridization method, the cycling probe method or the like.

The fluorescence intensity can be detected, for example, with a fluorometer. Moreover, in general, an apparatus which has both a PCR reaction unit (for example, a thermal cycler) and an optical unit (for example, a fluorometer) is used. Specific examples include commercial SmartCycler (product name, manufactured by Takara Bio Inc.), LightCycler (product name, manufactured by Roche Diagnostics), ABI PRISM7000 (product name, manufactured by Applied Biosystems) and the like.

### (Quantification Method of Target Nucleic Acid)

The method for quantifying a target nucleic acid of the invention is a method of generating an amplification product complementary to the target nucleic acid in the dried blood spot in a filter paper, detecting the amplification product by optical means and quantifying the amplification product. By counting the PCR cycle number at which the amount of the amplification product has reached a certain amount, the target nucleic acid contained in the dried blood spot in a filter paper can be quantified in the method. Moreover, as in the Examples described below, the quantification can be conducted by calculating the copy number of 1 µL of whole blood in the dried blood spot in a filter paper from a calibration curve of the Cq values calculated from the standards.

The invention relates to a method for detecting a target nucleic acid in a dried blood spot in a filter paper by real-time PCR, including the steps of (A) to (D) below.
(A) A step of adding the dried blood spot in a filter paper to a PCR reaction tube, wherein the filter paper is a circular punched piece with a diameter of 1.2 mm to 2.0 mm or a punched piece containing whole blood in an amount of 0.95 v/v% to 6.6 v/v% based on the total amount of the reaction solution.
(B) A step of adding 20 to 50 µL of a PCR reagent to the PCR reaction tube.
(C) A step of performing PCR reaction in the tube which contains the PCR reagent and the filter paper and which is sealed with a cap.
(D) A step of sequentially and optically detecting the target nucleic acid amplified by the PCR reaction.

Here, either one of the (A) step and the (B) step may be conducted first, or the steps may be conducted simultaneously. However, the step of (B) is desirably conducted after the step of (A). By conducting (A) first, the filter paper can be more certainly placed at the bottom of the tube with the reagent solution even when the filter paper adheres to the tube wall or the like. To ensure that the punched piece is placed at the bottom of the tube before starting the PCR reaction, centrifugation or the like is also desirable.

In this manner, the invention is characterized by subjecting the blood spot in a filter paper directly to real-time PCR without pretreatment such as separation and purification from the dried blood spot in a filter paper. A sample which has not been subjected to such pretreatment generally contains substances which inhibit the DNA amplification reaction. The substances which inhibit the DNA amplification reaction are hemoglobin contained in the blood, precipitates of blood components generated by thermal denaturation during the PCR reaction and the like.

Thus, when the PCR reaction is performed without any pretreatment, it is expected that the PCR reaction is affected by the interfering substances. However, according to the invention, as described above, by adjusting the size of the punched piece or the whole blood amount contained in the punched piece and the amount of the PCR reaction reagent in the certain ranges, placing the punched piece at the bottom of the tube and performing the PCR reaction after sealing the tube with the cap, generation of bubbles due to the temperature changes can be prevented, and vibration can be prevented without lifting the paper piece. As a result, the excessive elution of interfering substances from the punched piece can be prevented, and optical detection and quantification of the target nucleic acid contained in the punched piece directly by real-time PCR have been enabled without conducting any complicated pretreatment.

### (Target Nucleic Acid)

The nucleic acid as the target to be detected by real-time PCR in the invention is not particularly restricted, and in addition to whole blood-derived DNA and RNA, a foreign nucleic acid introduced to the whole blood by a virus or the like and the like are all included.

In particular, the target nucleic acid of the invention is preferably, for example, a nucleic acid derived from the whole blood of a newborn and is a nucleic acid contained in a gene fragment of TRECs (T-cell receptor excision circles) or KRECs (kappa-deleting recombination excision circles) or a gene such as *SMN1* (Survival Motor Neuron 1) and *SMN2* (Survival Motor Neuron 2).

### (Standard/Kit)

The standard (also called a calibrator) of the invention refers to a standard substance used for a method for quantifying a target nucleic acid by the method for detecting a target nucleic acid. The standard of the invention is characterized by containing an artificial nucleic acid including the sequence of the target nucleic acid and by not being contained in the dried blood spot in a filter paper.

Regarding the form of the standard, in a possible method, the standard may be added to the dried blood spot in a filter paper and used for quantification to offset the influence of the dried blood spot in a filter paper, but the accuracy and the reproducibility may be deteriorated due to the variation in the degree of influence due to the variation in the components extracted from the dried blood spot in a filter paper or in the elution of the standard from the dried blood spot in a filter paper and the like.

In this regard, the standard of the invention of the present application is intentionally provided in a form of not being contained in the dried blood spot in a filter paper and is a standard present in the form of, for example, solution state, a freeze-dried product or the like. The standard of the invention is in such a form and thus can be dissolved directly in the reaction solution and used, which achieves accurate quantification.

The invention can also provide the use of a quantification kit for a target nucleic acid including such a standard.

### EXAMPLES

The invention is explained in detail below by Examples, but the invention is not limited to the following Examples.

The Examples are examples in which punched pieces were taken out from dried blood spots in filter papers and subjected directly to PCR reaction and in which the amplification products were optically detected by real-time PCR.

### [Example 1] TREC/KREC Gene Quantification Systems

### (a) Test Materials

### (a-1) TREC, KREC and RNase P Amplification Primers

As primers for amplification of TRECs, KRECs and an internal standard gene (RNase P), the following primers were synthesized by Sigma-Aldrich Co. LLC and used.
TREC forward primer
   5'-CCCTTTCAACCATGCTGACA-3' (SEQ ID NO: 1)
TREC reverse primer
   5'-GTGCCAGCTGCAGGGTTTAG-3' (SEQ ID NO: 2)
KREC forward primer
   5'-CAGCTCAGCGCCCATTACG-3' (SEQ ID NO: 3)
KREC reverse primer
   5'-TGGGACTCCAGGAGCCAG-3' (SEQ ID NO: 4)
RNase P forward primer
   5'-GCGGAGGGAAGCTCATCA-3' (SEQ ID NO: 5)
RNase P reverse primer
   5'-GTCTGACCTCGCGCGGA-3' (SEQ ID NO: 6)

### (a-2) TREC, KREC and RNase P Detection Probes

As probes for observing the PCR amplification of TRECs, KRECs and RNase P, the following detection probes which were fluorescently labeled were synthesized and produced by Primetech Corporation and used.

TREC detection probe
   5'-(Quasar670)-CCTCTGGTTTTTGTAAAGGTGCCCACT-(BHQ3)-3' (the nucleotide sequence part is SEQ ID NO: 7)
KREC detection probe
   5'-(ROX)-TCTGCACGGGCAGCAGGTTGG-(BHQ2)-3' (the nucleotide sequence part is SEQ ID NO: 8)
RNase P detection probe
   5'-(FAM)-CCACGAGCTGAGTGCGTCCTG-(BHQ1)-3' (the nucleotide sequence part is SEQ ID NO: 9)

### (a-3) Plasmids

For quantifying the PCR amplification products, plasmids having the partial sequences of TREC, KREC and RNase P genes shown below incorporated in a vector were synthesized by Eurofins Scientific SE and used as standards for calibration curves.
Partial TREC Sequence (SEQ ID NO: 10)
Partial KREC Sequence (SEQ ID NO: 11)
Partial RNase P Sequence (SEQ ID NO: 12)

### (b) PCR Reagent

The composition of the PCR reagent is shown below.
PCR buffer (Ampdirect Plus; Shimadzu Corporation)
0.25 µM (final concentration) TREC forward primer
0.25 µM (final concentration) TREC reverse primer
0.125 µM (final concentration) KREC forward primer
0.125 µM (final concentration) KREC reverse primer
0.125 µM (final concentration) RNase P forward primer
0.125 µM (final concentration) RNase P reverse primer
0.135 µM (final concentration) TREC detection probe
0.135 µM (final concentration) KREC detection probe
0.135 µM (final concentration) RNase P detection probe
0.03 U/µL BIOTAQ HSDNA polymerase

### (c) PCR Reagents Containing Standards for Calibration Curves

PCR reagents containing the standards below (dilution series; STD1 to 4) were prepared by adding the plasmids to the PCR reagent. The values in the brackets are the copy numbers of the genes per one PCR reaction. The reagents were used in the solution state without adding to a dried blood spot in a filter paper.
STD1 (TRECs 10,000 copy/reaction, KRECs 10,000 copy/reaction, RNase P 100,000 copy/reaction)
STD2 (TRECs 1,000 copy/reaction, KRECs 1,000 copy/reaction, RNase P 10,000 copy/reaction)
STD3 (TRECs 100 copy/reaction, KRECs 100 copy/reaction, RNase P 1,000 copy/reaction)
STD4 (TRECs 10 copy/reaction, KRECs 10 copy/reaction, RNase P 100 copy/reaction)

### (d) Control Dried Blood Spot in a Filter Papers

After adding the TREC plasmid and the KREC plasmid to swine whole blood each at 12.5 copy/µL and further adding genomic DNA extracted from cell line K562 (derived from chronic myelogenous leukemia cells; provided from National Institutes of Biomedical Innovation, Health and Nutrition, Cell Bank) at 13.5 ng/µL, 40 µL thereof was blotted on a filter paper for blood collection (Advantec) and directly dried.

### (e) Conditions for PCR Reaction

(i) 95°C: 15 minutes
(ii) 95°C: 15 seconds, 63°C: 80 seconds (45 cycles)
(iii) 37°C: 5 minutes

### (f) Real-Time PCR Measurement

### (f-1) Measurement Method

The measurement was made by the following procedures.
(i) From the control dried blood spots in filter papers, circular punched pieces with diameters of 1.2 mm, 1.5 mm, 1.8 mm, 2.0 mm and 3.0 mm were taken out using a puncher.
(ii) To PCR reaction tubes (manufactured by Roche, Inc.: LightCycler (registered trademark) 8-Tube Strips, made of polypropylene), the punched pieces were introduced.
(iii) After adding 20 µL, 30 µL, 40 µL or 50 µL of the PCR reagents to the tubes, the tubes were sealed with caps (included as a set with the tubes).
(iv) The PCR reagents containing the standards for calibration curves according to the addition amounts were also dispensed to tubes.
(v) Using a thermal cycler (LightCycler96; Roche, Inc.), triplex real-time PCR measurement for TRECs, KRECs and RNase P was conducted.
(vi) The combinations were measured each four times. The copy numbers per 1 µL whole blood in the punched pieces were calculated from the calibration curves of the Cq values calculated from the standards, and the average values of the copy numbers of the four measurements were calculated. The values obtained by dividing the average values by the set values were regarded as the recovery rates.

### (f-2) Measurement Results

The results are shown in Tables 1 to 3. Regarding the difference in the size of the punched piece, the reproducibility of the amplification and the recovery rates were excellent in the case of diameters of 1.5 mm to 2.0 mm. With the diameter of 1.2 mm, amplification was not caused in some cases, but the recovery rates were excellent. On the other hand, in the case of the diameter of 3.0 mm, measurement could not be made in some cases because an amplification curve could not be drawn (the parts with hyphen (-) or shown in bold in the tables). It is believed that the causes were that the punched piece had a large size and thus did not reach the bottom of the tube and that the paper piece thus blocked the light path, which prevented the light from reaching the reaction solution and affected the fluorescence detection.

**[Table 1]**

| Table 1 TREC Measurement Results | | | | | | | | |
|---|---|---|---|---|---|---|---|---|
| Amount of PCR Reaction Solution | Size of Punched Piece | TREC (copy/µL W.B.) | N=1 | N=2 | N=3 | N=4 | Average (copy/µL W.B.) | Recovery Rate % |
| 20µL | 1.2mm | 12.5 | 5.6 | 22.3 | 8.6 | 10.3 | 11.7 | 94 |
| | 1.5mm | 12.5 | 16.8 | 23.5 | 11.5 | 22.0. | 18.4 | 147 |
| | 1.8mm | 12.5 | 17.6 | 4.8 | 13.3 | 14.5 | 12.5 | 100 |
| | 2.0mm | 12.5 | 22.8 | 14.3 | 10.8 | 16.5 | 16.1 | 129 |
| | 3.0mm | 12.5 | - | - | - | - | - | - |
| 30µL | 1.2mm | 12.5 | 8.1 | 3.2 | 17.4 | 18.4 | 11.8 | 94 |
| | 1.5mm | 12.5 | 26.1 | 20.1 | 10.2 | 20.8 | 19.3 | 154 |
| | 1.8mm | 12.5 | 6.1 | 6.6 | 16.8 | 6.4 | 8.9 | 72 |
| | 2.0mm | 12.5 | 5.5 | 14.3 | 20.0 | 2.4 | 10.6 | 85 |
| | 3.0mm | 12.5 | - | - | - | - | - | - |
| 40µL | 1.2mm | 12.5 | - | 7.1 | 6.0 | 19.9 | 11.0 | 88 |
| | 1.5mm | 12.5 | 10.2 | 5.6 | 12.0 | 19.8 | 11.9 | 95 |
| | 1.8mm | 12.5 | 23.3 | 14.7 | 9.2 | 10.1 | 14.3 | 115 |
| | 2.0mm | 12.5 | 10.5 | 9.1 | 8.4 | 7.1 | 8.8 | 70 |
| | 3.0mm | 12.5 | 44.8 | - | - | 33.8 | **39.3** | **314** |
| 50µL | 1.2mm | 12.5 | 23.0 | 9.9 | 11.5 | 26.1 | 17.6 | 141 |
| | 1.5mm | 12.5 | 5.6 | 20.2 | 16.5 | 11.8 | 13.5 | 108 |
| | 1.8mm | 12.5 | 44.6 | 6.8 | 11.3 | 8.0 | 17.7 | 141 |
| | 2.0mm | 12.5 | 6.7 | 20.8 | 19.4 | 11.0 | 14.5 | 116 |
| | 3.0mm | 12.5 | 65.2 | 220.2 | 10.8 | - | **98.7** | **790** |

**[Table 2]**

| Table 2 KREC Measurement Results | | | | | | | | |
|---|---|---|---|---|---|---|---|---|
| Amount of PCR Reaction Solution | Size of Punched Piece | KREC (copy/µL W.B.) | N=1 | N=2 | N=3 | N=4 | Average (copy/µL W.B.) | Recovery Rate % |
| 20µL | 1.2mm | 12.5 | - | 7.8 | 21.9 | 9.9 | 13.2 | 106 |
| | 1.5mm | 12.5 | 6.5 | 20.3 | 9.0 | 20.8 | 14.2 | 113 |
| | 1.8mm | 12.5 | 22.2 | 10.0 | 14.7 | 10.1 | 14.2 | 114 |
| | 2.0mm | 12.5 | 10.0 | 17.2 | 7.6 | 13.0 | 11.9 | 96 |
| | 3.0mm | 12.5 | - | - | - | - | - | - |
| 30µL | 1.2mm | 12.5 | 3.1 | 2.4 | 13.8 | 36.7 | 14.0 | 112 |
| | 1.5mm | 12.5 | 9.4 | 5.7 | 11.1 | 8.2 | 8.6 | 69 |
| | 1.8mm | 12.5 | 5.6 | 8.1 | 6.7 | 6.1 | 6.6 | 53 |
| | 2.0mm | 12.5 | 14.2 | 7.6 | 14.2 | 14.5 | 12.6 | 101 |
| | 3.0mm | 12.5 | 56.3 | - | - | - | **56.3** | **450** |
| 40µL | 1.2mm | 12.5 | 15.6 | 14.1 | - | 14.7 | 14.8 | 119 |
| | 1.5mm | 12.5 | 13.1 | 5.3 | 11.9 | 6.7 | 9.3 | 74 |
| | 1.8mm | 12.5 | 9.3 | 28.4 | 4.5 | 15.3 | 14.4 | 115 |
| | 2.0mm | 12.5 | 8.7 | 14.8 | 11.7 | 17.0 | 13.0 | 104 |
| | 3.0mm | 12.5 | - | - | 98.7 | 31.4 | **65.0** | **520** |
| 50µL | 1.2mm | 12.5 | 34.3 | 7.6 | 23.9 | 29.4 | 23.8 | 190 |
| | 1.5mm | 12.5 | 21.6 | 27.9 | 20.8 | 33.0 | 25.8 | 207 |
| | 1.8mm | 12.5 | 14.1 | 17.6 | 6.4 | 8.7 | 11.7 | 93 |
| | 2.0mm | 12.5 | 31.7 | 16.1 | 20.2 | 21.1 | 22.3 | 178 |
| | 3.0mm | 12.5 | 129.5 | 101.9 | 20.2 | 131.1 | 95.7 | 765 |

**[Table 3]**

| Table 3 RNase P Measurement Results (Reference) | | | | | | |
|---|---|---|---|---|---|---|
| Amount of PCR Reaction Solution | Size of Punched Piece | N=1 | N=2 | N=3 | N=4 | Average (copy/µL W.B.) |
| 20µL | 1.2mm | 795 | 770 | 958 | 643 | 791 |
| | 1.5mm | 972 | 700 | 723 | 657 | 763 |
| | 1.8mm | 517 | 497 | 603 | 527 | 536 |
| | 2.0mm | 1462 | 1337 | 469 | 1891 | 1290 |
| | 3.0mm | | - | - | - | - |
| 30µL | 1.2mm | 695 | 499 | 942 | 672 | 702 |
| | 1.5mm | 556 | 724 | 590 | 854 | 681 |
| | 1.8mm | 525 | 479 | 466 | 576 | 511 |
| | 2.0mm | 574 | 483 | 527 | 452 | 509 |
| | 3.0mm | - | - | - | - | - |
| 40µL | 1.2mm | 567 | 517 | 556 | 638 | 570 |
| | 1.5mm | 651 | 864 | 830 | 1350 | 924 |
| | 1.8mm | 525 | 615 | 2426 | 561 | 1032 |
| | 2.0mm | 489 | 512 | 1320 | 806 | 782 |
| | 3.0mm | - | - | - | - | - |
| 50µL | 1.2mm | 645 | 560 | 1336 | 593 | 783 |
| | 1.5mm | 670 | 649 | 522 | 911 | 688 |
| | 1.8mm | 635 | 531 | 580 | 664 | 602 |
| | 2.0mm | 618 | 551 | 681 | 668 | 629 |
| | 3.0mm | - | - | 1283 | - | 1283 |

### INDUSTRIAL APPLICABILITY

According to the invention, specific and rapid detection and quantification of a slight amount of a target nucleic acid contained in a dried blood spot in a filter paper by real-time PCR are enabled.

## Claims

1. A method for detecting a target nucleic acid contained in a dried blood spot in a filter paper by real-time PCR, including the steps of (A) to (D) below:
(A) a step of adding the dried blood spot in a filter paper to a PCR reaction tube, wherein the filter paper is a circular punched piece with a diameter of 1.2 mm to 2.0 mm or a punched piece containing whole blood in an amount of 0.95 v/v% to 6.6 v/v% based on the total amount of the reaction solution;
(B) a step of adding 20 to 50 µL of a PCR reagent to the PCR reaction tube;
(C) a step of performing PCR reaction in the tube which contains the PCR reagent and the dried blood spot in a filter paper and which is sealed with a cap; and
(D) a step of sequentially and optically detecting the target nucleic acid amplified by the PCR reaction,
wherein the blood spot in the filter paper is subjected directly to real-time PCR without pre-treatment.

2. The method for detecting a target nucleic acid according to claim 1, wherein the step of (B) is conducted after the step of (A).

3. The method for detecting a target nucleic acid according to claim 1 or 2, wherein the PCR reaction tube is a 96-well plate for PCR reaction or a tube in an eight-tube strip.

4. The method for detecting a target nucleic acid according to any of claims 1 to 3, wherein the PCR reagent contains at least a primer, a polymerase, dNTPs and an intercalator or a fluorescently labeled probe.

5. The method for detecting a target nucleic acid according to claim 4, wherein the amplified target nucleic acid is detected through detection of fluorescence emitted by irradiation of the intercalator with an excitation light.

6. The method for detecting a target nucleic acid according to claim 4, wherein the fluorescently labeled probe has a fluorescent substance and a quencher and includes a partial sequence complementary to a template of the nucleic acid amplification reaction, and the amplified target nucleic acid is detected through detection of fluorescence emitted by irradiation of the fluorescent substance with an excitation light.

7. The method for detecting a target nucleic acid according to any of claims 1 to 6, wherein the target nucleic acid is one or more gene fragments and/or genes selected from the group consisting of TRECs, KRECs and *SMN1.*

8. A method for quantifying a target nucleic acid by the method for detecting a target nucleic acid by real-time PCR according to any of claims 1 to 7, wherein the target nucleic acid is quantified using a standard below;
(1) a standard which contains an artificial nucleic acid including the sequence of the target nucleic acid and which is not contained in the dried blood spot in a filter paper, wherein the standard is dissolved directly in a reaction container and used.

9. Use of a quantification kit in a method for quantifying a target nucleic acid by the method for detecting a target nucleic acid by real-time PCR according to any of claims 1 to 7, wherein the quantification kit includes at least the following:
(1) a standard which contains an artificial nucleic acid including the sequence of the target nucleic acid and which is not contained in the dried blood spot in a filter paper, wherein the standard is dissolved directly in a reaction container and used.

## Patentansprüche

1. Verfahren zum Detektieren einer in einem getrockneten Blutfleck in einem Filterpapier enthaltenen Zielnukleinsäure durch Echtzeit-PCR, welches die Schritte (A) bis (D) unten einschließt:
(A) einen Schritt des Zugebens des getrockneten Blutflecks in einem Filterpapier zu einem PCR-Reaktionsröhrchen, wobei das Filterpapier ein kreisförmiges ausgestanztes Stück mit einem Durchmesser von 1,2 mm bis 2,0 mm oder ein ausgestanztes Stück ist, welches Vollblut in einer Menge von 0,95% v/v bis 6,6% v/v, basierend auf der Gesamtmenge der Reaktionslösung, enthält;
(B) einen Schritt des Zugebens von 20 bis 50 µl eines PCR-Reagens zu dem PCR-Reaktionsröhrchen;
(C) einen Schritt des Durchführens einer PCR-Reaktion in dem Röhrchen, welches das PCR-Reagens und den getrockneten Blutfleck in einem Filterpapier enthält und welches mit einer Kappe verschlossen ist; und
(D) einen Schritt des sequentiellen und optischen Detektierens der durch die PCR-Reaktion amplifizierten Zielnukleinsäure,
wobei der Blutfleck in dem Filterpapier ohne Vorbehandlung direkt Echtzeit-PCR unterworfen wird.

2. Verfahren zum Detektieren einer Zielnukleinsäure gemäß Anspruch 1, wobei Schritt (B) nach Schritt (A) durchgeführt wird.

3. Verfahren zum Detektieren einer Zielnukleinsäure gemäß Anspruch 1 oder 2, wobei das PCR-Reaktionsröhrchen eine 96-Well-Platte für PCR-Reaktion oder ein Röhrchen in einem acht-Röhrchen-Streifen ist.

4. Verfahren zum Detektieren einer Zielnukleinsäure gemäß irgendeinem der Ansprüche 1 bis 3, wobei das PCR-Reagens wenigstens einen Primer, eine Polymerase, dNTPs und einen Interkalator oder eine fluoreszierend markierte Sonde enthält.

5. Verfahren zum Detektieren einer Zielnukleinsäure gemäß Anspruch 4, wobei die amplifizierte Zielnukleinsäure durch Detektion von Fluoreszenz, emittiert durch Bestrahlen des Interkalators mit einem Anregungslicht, detektiert wird.

6. Verfahren zum Detektieren einer Zielnukleinsäure gemäß Anspruch 4, wobei die fluoreszierend markierte Sonde eine fluoreszierende Substanz und einen Quencher aufweist und eine partielle Sequenz einschließt, die komplementär zu einem Template der Nukleinsäureamplifikationsreaktion ist, und die amplifizierte Zielnukleinsäure durch Detektion von Fluoreszenz, emittiert durch Bestrahlen der fluoreszierenden Substanz mit einem Anregungslicht, detektiert wird.

7. Verfahren zum Detektieren einer Zielnukleinsäure gemäß irgendeinem der Ansprüche 1 bis 6, wobei die Zielnukleinsäure ein oder mehrere Genfragmente und/oder Gene ist, ausgewählt aus der Gruppe, bestehend aus TRECs, KRECs und *SMN1.*

8. Verfahren zum Quantifizieren einer Zielnukleinsäure durch das Verfahren zum Detektieren einer Zielnukleinsäure durch Echtzeit-PCR gemäß irgendeinem der Ansprüche 1 bis 7, wobei die Zielnukleinsäure unter Verwendung eines Standards unten quantifiziert wird;
(1) eines Standards, welcher eine artifizielle Nukleinsäure enthält, die die Sequenz der Zielnukleinsäure einschließt, und nicht in dem getrockneten Blutfleck in einem Filterpapier enthalten ist, wobei der Standard direkt in einem Reaktionsbehälter gelöst und verwendet wird.

9. Verwendung eines Quantifizierungskits in einem Verfahren zum Quantifizieren einer Zielnukleinsäure durch das Verfahren zum Detektieren einer Zielnukleinsäure durch Echtzeit-PCR gemäß irgendeinem der Ansprüche 1 bis 7, wobei das Quantifizierungskit wenigstens folgendes einschließt:
(1) einen Standard, welcher eine artifizielle Nukleinsäure enthält, die die Sequenz der Zielnukleinsäure einschließt, und nicht in dem getrockneten Blutfleck in einem Filterpapier enthalten ist, wobei der Standard direkt in einem Reaktionsbehälter gelöst und verwendet wird.

## Revendications

1. Procédé de détection d'un acide nucléique cible contenu dans une goutte de sang séchée sur un papier-filtre par PCR en temps réel, incluant les étapes (A) à (D) ci-dessous :
(A) une étape consistant à ajouter la goutte de sang séchée sur un papier-filtre à un tube de réaction de PCR, dans lequel le papier-filtre est une pièce poinçonnée circulaire avec un diamètre de 1,2 mm à 2,0 mm ou une pièce poinçonnée contenant du sang total dans une quantité de 0,95 v/v % à 6,6 v/v % sur la base de la quantité totale de la solution de réaction ;
(B) une étape consistant à ajouter 20 à 50 µL d'un réactif de PCR au tube de réaction de PCR ;
(C) une étape consistant à effectuer une réaction de PCR dans le tube qui contient le réactif de PCR et la goutte de sang séchée sur un papier-filtre et qui est scellé avec un capuchon ; et
(D) une étape consistant à détecter séquentiellement et optiquement l'acide nucléique cible amplifié par la réaction de PCR,
dans lequel la goutte de sang sur le papier-filtre est soumise directement à une PCR en temps réel sans prétraitement.

2. Procédé de détection d'un acide nucléique cible selon la revendication 1, dans lequel l'étape (B) est conduite après l'étape (A).

3. Procédé de détection d'un acide nucléique cible selon la revendication 1 ou la revendication 2, dans lequel le tube de réaction de PCR est une plaque de 96 puits pour réaction de PCR ou un tube d'une barrette de huit tubes.

4. Procédé de détection d'un acide nucléique cible selon l'une quelconque des revendications 1 à 3, dans lequel le réactif de PCR contient au moins une amorce, une polymérase, des dNTP et un agent intercalant ou une sonde marquée de manière fluorescente.

5. Procédé de détection d'un acide nucléique cible selon la revendication 4, dans lequel l'acide nucléique cible amplifié est détecté par détection d'une fluorescence émise par irradiation de l'agent intercalant avec une lumière d'excitation.

6. Procédé de détection d'un acide nucléique cible selon la revendication 4, dans lequel la sonde marquée de manière fluorescente présente une substance fluorescente et un extincteur et inclut une séquence partielle complémentaire à une matrice de la réaction d'amplification d'acide nucléique, et l'acide nucléique cible amplifié est détecté par détection d'une fluorescence émise par irradiation de la substance fluorescente avec une lumière d'excitation.

7. Procédé de détection d'un acide nucléique cible selon l'une quelconque des revendications 1 à 6, dans lequel l'acide nucléique cible est un ou plusieurs fragments géniques et/ou gènes sélectionnés parmi le groupe constitué de TRECs, KRECs et *SMN1.*

8. Procédé de quantification d'un acide nucléique cible par le procédé de détection d'un acide nucléique cible par PCR en temps réel selon l'une quelconque des revendications 1 à 7, dans lequel l'acide nucléique cible est quantifié en utilisant un étalon ci-dessous ;
(1) un étalon qui contient un acide nucléique artificiel incluant la séquence de l'acide nucléique cible et qui n'est pas contenu dans la goutte de sang séchée sur un papier-filtre, dans lequel l'étalon est dissous directement dans un récipient de réaction et utilisé.

9. Utilisation d'un kit de quantification dans un procédé de quantification d'un acide nucléique cible par le procédé de détection d'un acide nucléique cible par PCR en temps réel selon l'une quelconque des revendications 1 à 7, dans lequel le kit de quantification inclut au moins ce qui suit :
(1) un étalon qui contient un acide nucléique artificiel incluant la séquence de l'acide nucléique cible et qui n'est pas contenu dans la goutte de sang séchée sur un papier-filtre, dans lequel l'étalon est dissous directement dans un récipient de réaction et utilisé.


### SEQUENCE LISTING

<110> SEKISUI MEDICAL CO., LTD.
<120> Nucleic Acid Detection Method by Real-Time PCR
<130> 20P00983WO00
<160> 12
<170> PatentIn version 3.5
<210> 1
   <211> 20
   <212> DNA
   <218> Artificial Sequence
<220>
   <223> primer
<400> 1
   ccctttcaac catgctgaca 20
<210> 2
   <211> 20
   <212> DNA
   <218> Artificial Sequence
<220>
   <223> primer
<400> 2
   gtgccagctg cagggtttag 20
<210> 3
   <211> 19
   <212> DNA
   <218> Artificial Sequence
<220>
   <223> primer
<400> 3
   cagctcagcg cccattacg 19
<210> 4
   <211> 18
   <212> DNA
   <218> Artificial Sequence
<220>
   <223> primer
<400> 4
   tgggactcca ggagccag 18
<210> 5
   <211> 18
   <212> DNA
   <218> Artificial Sequence
<220>
   <223> primer
<400> 5
   gcggagggaa gctcatca 18
<210> 6
   <211> 17
   <212> DNA
   <218> Artificial Sequence
<220>
   <223> primer
<400> 6
   gtctgacctc gcgcgga 17
<210> 7
   <211> 27
   <212> DNA
   <218> Artificial Sequence
<220>
   <223> probe
<400> 7
   cctctggttt ttgtaaaggt gcccact 27
<210> 8
   <211> 21
   <212> DNA
   <218> Artificial Sequence
<220>
   <223> probe
<400> 8
   tctgcacggg cagcaggttg g 21
<210> 9
   <211> 21
   <212> DNA
   <218> Artificial Sequence
<220>
   <223> probe
<400> 9
   ccacgagctg agtgcgtcct g 21
<210> 10
   <211> 503
   <212> DNA
   <218> Homo sapiens
<400> 10
   gactgaattc ggctctgtct agtgtgataa cattttgtta tcttattcat tgtcttcatc      60
   cctgaaatac actctgctct ctcctatctc tgctctgaaa ggcagaaaga gggcagccct     120
   ctccaaggca aaatggggct cctgtgggga acagaggggt gcctctgtca acaaaggtga     180
   tgccacatcc ctttcaacca tgctgacacc tctggttttt gtaaaggtgc ccactcctgt     240
   gcacggtgat gcataggcac ctgcaccccg tgcctaaacc ctgcagctgg cacgggccct     300
   gtctgctctt cattcaccgt tctcacgagt tgcaataagt tcagccctcc atgtcacact     360
   gtgttttcca tcctggggag tgtttcacag ctatcccaag ccccacgctg acgaatcacg     420
   gccgaaaaca cactctgatg ccagcacaga ccacggagca aatgtcagac aagatcagcc     480
   tcggaaaagt gaggaattca gtc                                             503
<210> 11
   <211> 421
   <212> DNA
   <218> Homo sapiens
<400> 11
   gctttaaatt atttacaatc ccctaatgga aattttcact aatgagatat cataatgaat 60
   gtgaatttta tttctgaaat ctctaataaa tcagtcttct ccctggtttt cccagctcag 120
   cgcccattac gtttctgttc tctttccctt agtggcatta tttgtatcac tgtgcatcag 180
   cacagtgtgc gctgccaacc tgctgcccgt gcagaaactc tagggtaaga gctggctcct 240
   ggagtcccac ccaggctgcg tgtccctcac agtctgctct gtgtctatgt gtgtgtgttg 300
   gggggatatt attggacaat tcaagggagg ctcagtgagg aggaaggaaa atttcatgta 360
   attctttctg gtttgtcttt ctcaaccccc tcctcctgcc tactccttaa ctgagagagt 420
   g 421
<210> 12
   <211> 341
   <212> DNA
   <218> Homo sapiens
<400> 12
   atagggcgga gggaagctca tcagtggggc cacgagctga gtgcgtcctg tcactccact 60
   cccatgtccc ttgggaaggt ctgagactag ggccagaggc ggccctaaca gggctctccc     120
   tgagcttcgg ggaggtgagt tcccagagaa cggggctccg cgcgaggtca gactgggcag     180
   gagatgccgt ggaccccgcc cttcggggag gggcccggcg gatgcctcct ttgccggagc     240
   ttggaacaga ctcacggcca gcgaagtgag ttcaatggct gaggtgaggt accccgcagg     300
   ggacctcata acccaattca gactactctc ctccgcccat t                    341